# EUROPEAN PATENT SPECIFICATION

(11) **EP 1 532 972 B1**
(45) Date of publication and mention of the grant of the patent: **27.06.2007**
(21) Application number: 04077817.7
(22) Date of filing: 13.10.2004
(51) Int. Cl.: A61K 8/26, A61K 8/28, A61K 8/81, A61Q 15/00

(54) **Antiperspirant compositions**
Schweisshemmende Zusammensetzungen
Compositions antitranspirantes

(30) Priority: 11.11.2003 GB 0326181
(43) Date of publication of application: 25.05.2005
(73) Proprietor: UNILEVER PLC, London EC4P 4BQ (GB); UNILEVER N.V., 3013 AL Rotterdam (NL)
(72) Inventor: Cropper, Martin Peter, Unilever R&D Sunlight, Wirral Merseyside CH63 3JW (GB)
(74) Representative: Whaley, Christopher

(56) References cited:
- EP-A- 0 478 327
- EP-A- 0 584 877
- WO-A-02/43687
- WO-A-02/49590

## Description

### FIELD OF INVENTION

This invention relates to the field of cosmetic compositions giving an antiperspirancy and deodorancy benefit. More specifically, it relates to aqueous antiperspirant compositions comprising a suspension of an antiperspirant salt and a polymer having Bronsted acid groups.

### BACKGROUND OF INVENTION

Cosmetic antiperspirant compositions are well known. Typical antiperspirant compositions comprise an antiperspirant salt, such as an astringent aluminium or aluminium/zirconium salt, in combination with a cosmetically suitable vehicle. Such cosmetic antiperspirant compositions are available in a variety of product forms, for example as sticks, creams, soft-solids, roll-on lotions, aerosols, pump sprays and squeeze sprays.

Whilst such compositions provide a degree of antiperspirancy and malodour reduction, there can be problems associated with their use and there is always a desire for improved performance. A problem encountered by some people is that the application of certain antiperspirant compositions, in particular those having a high level of astringent antiperspirant salt, leads to skin irritation. Other problems include formulation difficulties with the high levels of active ingredients sometimes required and incompatibility between certain components of the composition. It is an objective of the present invention to reduce perspiration and to achieve excellent protection from body malodour without the use of high concentrations of conventional antiperspirant or deodorant agents.

The above problems have been addressed in a number of ways in the past, examples including the use of certain polymers as antiperspirant actives. WO 93/24105 (Tranner) describes the use of particular water-insoluble film-forming polymers, with conventional antiperspirant salts being non-essential, optional components in the compositions of the invention. The examples given that include antiperspirant salt also comprise co-polymers of octylacrylamide/acrylates or PVP/acrylates. No reference is made to interactions between the antiperspirant salts and the polymers. References to film-forming polymers are also made in JP 2290810 (Nakagawa et al) and WO 95/27473 (Causton and Baines). An alternative approach is described in EP 701812 (Abrutyn et al), where porous polymer beads are claimed to be capable of absorbing sweat components.

Polymers have also been used to enhance the performance of antiperspirant salts by increasing the residual amount of antiperspirant salt on the skin. Thus, EP 222580 (Klein and Sykes) describes the use of dimethyldiallyl ammonium chloride (DMDAAC) polymers for this purpose.

The use of DMDAAC/acrylic acid-type co-polymers to thicken personal care products is described in EP 266,111 (Boothe et al) and EP 478,327 (Melby and Boothe). The latter of these patents discusses the thickening of metal-containing aqueous compositions by said co-polymers.

Aqueous compositions comprising an acrylic acid containing polymer and an antiperspirant salt are described in WO 98/50005 and WO 98/48768 (Ron et al). In these patents, the proposed invention relates to the reverse thermal viscosifying benefit of the polymer.

US 5,194,262 and US 5,271,934 (Goldberg et al) describe antiperspirant compositions containing microcapsules comprising an antiperspirant salt encapsulated within a water-soluble shell possessing a bioadhesive. Polyacrylic acid is disclosed as a possible component of both the water-soluble shell and the bioadhesive.

WO 02/49590 (Smith et al) discloses antiperspirant compositions comprising an antiperspirant salt and a polymer having Bronsted acid groups which, in the presence of water, acts as a co-gellant for the antiperspirant salt; however, unlike the case in the present invention, the antiperspirant salt and the polymer are kept physically separate prior to application.

### SUMMARY OF INVENTION

We have discovered that antiperspirant compositions having superior performance and stability may be prepared by suspending an antiperspirant salt and a polymer having Bronsted acid groups in an aqueous phase, provided that the particle size of the composition is kept sufficient low.

Thus, according to a first aspect of the present invention, there is provided an antiperspirant composition comprising an aqueous phase, an antiperspirant salt and a polymer having Bronsted acid groups, characterised in that the antiperspirant salt and polymer are suspended in the aqueous phase and the composition has a Sauter mean particle size (D[3,2]) of 30 microns or less.

According to a second aspect of the invention, there is provided a cosmetic method of achieving an antiperspirancy and/or deodorancy benefit, said method comprising the application to the human body of a composition as described in the first aspect of the invention.

According to a third aspect of the present invention, there is provided a method for the manufacture of an antiperspirant composition, said method comprising the suspension in an aqueous phase of an antiperspirant salt and a polymer having Bronsted acid groups, the composition being sheared to give a Sauter mean particle size (D[3,2]) of 30 microns or less.

According to a fourth aspect of the present invention, there is provided a product comprising a composition as described in the first aspect of the invention and an applicator suitable for roll-on application of the composition.

### DETAILED DESCRIPTION OF THE INVENTION

The antiperspirant compositions of the present invention may give excellent results in terms of antiperspirancy and deodorancy. They are also surprisingly stable, dispense well, and have acceptable sensory properties.

The stability of the compositions of the invention is particularly surprising when one considers the strong interaction that occurs between the antiperspirant (AP) salt and the polymer having Bronsted acid groups in the presence of water. The interaction is chemical in nature and results in the production of particles of co-gel. These particles have to be relatively small, if they are to be formulated into a stable antiperspirant composition. The particles of co-gel have a Sauter mean particle size (D[3,2]) of 30 microns or less, in particular 25 microns or less, and especially 20 microns or less. Particle size measurements may be made using standard light scattering techniques, on instruments such as the Malvern Mastersizer.

Antiperspirant compositions according to the invention have a Sauter mean particle size (D[3,2]) of 30 microns or less, preferably 25 microns, and more preferably 20 microns or less. By processing the composition to have these relatively low particle sizes, one may gain benefits in terms of stability, dispensing, and/or sensory. The Sauter mean particle size (D[3,2]) of antiperspirant compositions according to the invention is a measurement made on the full composition, disperse phase components other than the co-gel particles (such as droplets of oil) being including in the determination.

### Polymers

The polymers used in the present invention have Bronsted acid groups that may interact with polyvalent hydrated metal salts, such salts resulting from the addition of AP salts to the aqueous environment of the composition. It is preferred that the polymer acts as a co-gellant for the AP salt. It is highly preferred that the polymer is water soluble. A simple test that may be used to determine whether or not a water soluble polymer is able to act as a co-gellant for a given AP salt consists of mixing aqueous solutions of the polymer and the AP salt and looking for an increase in viscosity.

The water solubility of the polymers used in the present invention, when measured at 37°C, is preferably 10g/l or greater, more preferably 50g/l or greater, and most preferably 100g/l or greater. It is desirable that the polymers form true solutions, rather than dispersions, in water; such true solutions typically having an absorbance of less than 0.2, preferably less than 0.1 (for a 1 cm pathlength at 600 nm) measured at 20°C using a Pharmacia Biotech Ultrospec 200 Spectrophotometer or similar instrument. It is also desirable that the polymer is water soluble at pH 7; the attainment of said pH generally requiring a certain amount of neutralisation of the Bronsted acid groups present.

The Bronsted acid groups in the polymer may be present in their protonated form or may be present in their neutralised form as salt groups. Both partially-neutralised and fully-neutralised acidic polymers may be employed. Suitable Bronsted acid groups include carboxylic acid groups, sulphonic acid groups, and phosphonic acid groups. Carboxylic acid groups are particularly preferred. Bronsted acid groups are preferably present at a concentration of greater than 0.1 mmole per gram of polymer, more preferably at a concentration of greater than 1.0 mmole per gram of polymer, and most preferably at a concentration of greater than 3.0 mmole per gram of polymer. These preferred levels relate to monobasic Bronsted acid groups and should be reduced *pro rata* for polybasic Bronsted acid groups. Latent Bronsted acid groups, such as anhydrides or other groups that generate Bronsted acid groups on addition to water, may also be present in the polymer used to prepare the compositions of the invention.

Preferred polymers are organic polymers, in particular, organic polymers possessing only limited positive charge - for example, organic polymers having less than 50 mole %, preferably less than 25 mole %, of positively-charged monomer units. Especially preferred organic polymers are nonionic and anionic polymers. Typical polymers possess carbon backbones, optionally interrupted by ester or amide links.

The acid value of a polymer is a widely used means of characterisation. Acid values generally express the acidity of a polymer in terms of the number of milligrams of potassium hydroxide base required to fully neutralise one gram of the polymer. Thus, the unit of measurement can be abbreviated to mg KOH/g.

Polymers used in the present invention may have acid values greater than 160. Preferred polymers have acid values greater than 320 or even greater than 450. Particularly preferred polymers have acid values greater than 580. These acid values are based on the polymer in its fully protonated state; that is to say, the actual in-use extent of neutralisation of the polymer is ignored in respect of the 'acid value'. Acid values may be measured experimentally or may be estimated theoretically. When using the latter method, acid anhydride groups present in a polymer should be counted as two acid groups, such anhydrides generally being hydrolysed to di-acids by potassium hydroxide.

The preferred carboxylic acid groups may be introduced into the polymer by inclusion of monomers such as acrylic acid, methacrylic acid, maleic acid, itaconic acid, crotonic acid, maleic anhydride, or itaconyl anhydride in the polymer.
When the only source of Bronsted acid groups are anhydride monomers, it is required that the anhydride groups are at least partially hydrolysed prior to incorporation of the polymer into the composition. Polymers comprising a mixture of any of the above acid and/or anhydride monomers may also be advantageously employed. Particularly preferred polymers are those derived, at least in part, from maleic acid and/or maleic anhydride monomers.

It is sometimes desirable to include other monomers in the polymer. Suitable monomers include methyl vinyl ether, C₁-C₈ alkyl acrylates and methacrylates, vinyl acetate, ethylene, and propylene. The inclusion of such monomers may aid polymer synthesis, ease handling and/or formulation of the polymer, and may improve the performance of the polymer as a co-gellant.

The molecular weight of the polymer is preferably in the range of 500 to 5,000,000, in particular 10,000 to 3,000,000 and especially 100,000 to 2,500,000. Selection of an appropriate molecular weight for the polymer may lead to benefits in terms of ease of formulation, product aesthetics (particularly product feel), and product performance.

The polymer is preferably incorporated into the composition in an amount of from 0.01% to 10% by weight, more preferably from 0.05% to 5% by weight, and most preferably from 1% to 3% by weight of said composition.

### Antiperspirant Salts

Antiperspirant salts for use herein are usually selected from astringent salts including, in particular, aluminium and mixed aluminium/zirconium salts, including both inorganic salts, salts with organic anions, and complexes. Preferred astringent salts are aluminium and aluminium/zirconium halides and halohydrate salts, such as chlorohydrates. Especially preferred are aluminium salts that exclude zirconium, these salts being most clearly enhanced in antiperspirancy by the presence of the polymer have Bronsted acid groups.

Aluminium halohydrates are usually defined by the general formula Al₂(OH)ₓQ_{y}.wH₂O in which Q represents chlorine, bromine or iodine, x is variable from 2 to 5 and x + y = 6 while wH₂O represents a variable amount of hydration. Especially effective aluminium halohydrate salts, known as activated aluminium chlorohydrates, are described in EP006,739 (Unilever PLC and NV). Some activated salts do not retain their enhanced activity in the presence of water but are useful in substantially anhydrous formulations, i.e. formulations that do not contain a distinct aqueous phase. Zirconium salts are usually defined by the general formula ZrO(OH)₂₋ₓQₓ.wH₂O in which Q represents chlorine, bromine or iodine; x is from about 1 to 2; w is from about 1 to 7; and x and w may both have non-integer values. Preferred are zirconyl oxyhalides, zirconium hyroxyhalides, and combinations thereof. Non-limiting examples of zirconium salts and processes for making them are described in Belgian Patent 825,146, Schmitz, issued August 4, 1975 and U.S. Patent 4,223,010 (Rubino).

The above aluminium and aluminium/zirconium salts may have co-ordinated and/or bound water in various quantities and/or may be present as polymeric species, mixtures or complexes.

Suitable aluminium-zirconium complexes often comprise a compound with a carboxylate group, for example an amino acid. Examples of suitable amino acids include tryptophan, phenylalanine, valine, methionine, alanine and, most preferably, glycine.

It is sometimes desirable to employ complexes of a combination of aluminium halohydrates and zirconium chlorohydrates together with amino acids such as glycine, which are disclosed in US 3,792,068 (Procter and Gamble Co.). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

Other actives that may be utilised include astringent titanium salts, for example those described in GB 2,299,506.

Antiperspirant salts are preferably incorporated into a composition in an amount of from 0.5-60%, particularly from 3 to 30% or 40% and especially from 5 or 10% to 30 or 35% of the weight of the composition.

The proportion of AP salt in a composition excludes the weight of any water of hydration present in the salt before its addition to the aqueous phase, but includes the weight of any complexing agent present.

The weight ratio of the AP salt to the polymer is preferably 50:1 or less, more preferably being from 25:1 to 1:10, and most preferably being from 10:1 and 1:5.

### The aqueous phase

The aqueous phase is normally a continuous phase and may comprise water-soluble species, in addition to water itself. However, water is typically the major component of this phase and normally accounts for 40% or greater, in particular 55% or greater, and especially 70% or greater of the composition. Other water soluble liquids may also be present; for example, short chain (C₁-C₄) alcohols, in particular monohydric alcohols such as ethanol or isopropanol, may be present, typically at a level of from 1 to 50%, in particular from 2 to 40%, and especially at from 5 to 30% by weight of the composition. In certain preferred embodiments, short chain (C₁-C₄) polyhydric alcohols are employed, suitable materials include glycerol and propylene glycol. Alternatively, longer chain, water soluble, polyhydric alcohols may be employed, such as dipropylene glycol or polyethylene glycol.

### Optional Additional Components

An emollient oil, typically accompanied by an emulsifier, is a highly preferred additional component of the compositions according to the invention. Such materials may enhance the sensory benefits delivered. The total level of emollient oil or oils used may be from 0.1% to 20% of the total weight of the composition. Suitable emollient oils include cyclomethicone, dimethicone, dimethiconol, isopropyl myristate, isopropyl palmitate, sunflower oil, C12-C15 alcohol benzoate, PPG-3 myristyl ether, octyl dodecanol, C7-C14 isoparaffins, di-isopropyl adipate, isosorbide laurate, PPG-14 butyl ether, PPG-15 stearyl ether, glycerol, propylene glycol, poly(ethylene glycol), hydrogenated polyisobutene, polydecene, phenyl trimethicone, dioctyl adipate, and hexamethyl disiloxane. The emollient oil is typically part of an oil-in-water emulsion composition having an aqueous continuous phase with emulsified oil droplets and particles of AP salt-polymer co-gel suspended therein. In such compositions, the total level of emollient oil or oils used is preferably from 0.2% to 5% of the total weight of the composition.

As indicated above, emulsifiers may be used in the compositions according to the invention. The total level of emulsifier or emulsifiers used may be from 0.1% to 10% of the total weight of the composition. Suitable emulsifiers include steareth-2, steareth-20, steareth-21, ceteareth-20, glyceryl stearate, cetyl alcohol, cetearyl alcohol, PEG-20 stearate, and dimethicone copolyol. Emulsifiers desirable in certain compositions of the invention are perfume solubilisers and wash-off agents. Examples of the former include PEG-hydrogenated castor oil, available from BASF in the Cremophor RH and CO ranges, preferably present at up to 1.5% by weight, more preferably 0.3 to 0.7% by weight. Examples of the latter include poly(oxyethylene) ethers.

A perfume or fragrance oil, is a highly preferred additional component of the compositions according to the invention. Suitable materials include conventional perfumes and so-called deo-perfumes, as described in EP 545,556 and other publications. Levels of incorporation are preferably up to 4% by weight, particularly from 0.1% to 2% by weight, and especially from 0.7% to 1.7% by weight of the composition.

A suspending agent may also be used to further enhance the stability of compositions according to the invention. The total amount of suspending agent or agents may be from 0.1 to 5% by weight of the total weight of the composition. Suitable suspending agents include quaternium-18 bentonite, quaternium-18 hectorite, silica (in particular, finely-divided or fumed silica), and propylene carbonate.

An additional component that can sometimes augment deodorancy performance is an organic anti-microbial agent. Levels of incorporation are preferably from 0.01% to 3%, more preferably from 0.03% to 0.5% by weight of the composition. Preferred organic anti-microbial agents are those that are more efficacious than ethanol. Antimicrobials that water soluble are also preferred. The preferred organic anti-microbials are also bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred anti-microbials are polyhexamethylene biguanide salts (also known as polyaminopropyl biguanide salts), an example being Cosmocil CQ available from Zeneca PLC; 2',4,4'-trichloro,2-hydroxydiphenyl ether (triclosan); and 3,7,11-trimethyldodeca-2,6,10-trienol (farnesol). Most preferred anti-microbials are transition metal chelators, in particular those that have a binding co-efficient for iron (III) of greater than 10²⁶, such as diethylenetriaminepentaacetic acid (DTPA) and salts thereof.

One or more of the following additional components may also be included in the compositions of the invention: colourants; preservatives, such as C₁-C₃ alkyl parabens; and irritation reducing agents, such as borage seed oil or ricinoleic acid.

### Product Forms

The composition of the invention is typically an emulsion, in particular an oil-in-water emulsion. The composition is preferably used as a roll-on product, together with an applicator suitable for roll-on application of the composition and typically comprising a roll-ball. Such roll-on compositions usually have an aqueous continuous phase and often an emulsified oil phase, in addition to suspended co-gel particles of AP salt-polymer. Other product forms are possible however, the composition of the invention possibly being a spray product, stick or soft solid, with the addition of appropriate adjuncts. Aerosol spray compositions may be prepared by adding a polar propellant such as dimethyl ether to an aqueous ethanol base. Stick and soft compositions may be prepared as water-in-oil emulsions, the AP salt-polymer co-gel particles being suspended in the water droplets. This later product structure may also be used for roll-ons and spray products.

### Methods of Manufacture

The method for the manufacture of antiperspirant compositions according to the invention comprises the suspension in an aqueous phase of an antiperspirant salt and a polymer having Bronsted acid groups, the composition being sheared to give a Sauter mean particle size (D[3,2]) of 30 microns or less. The shearing used typically produces co-gel particles of AP salt-polymer suspended in the aqueous phase. The method may also involve the emulsification of oil in the aqueous phase, producing an oil-in-water emulsion. In a preferred method of manufacture, the method comprises the addition of a suspension of co-gel particles of AP salt-polymer in water to an oil-in-water emulsion, the composition being sheared to give a Sauter mean particle size (D[3,2]) of 30 microns or less.

### EXAMPLES

In the following examples, comparative examples are indicated by letters and examples according to the invention are indicated by numbers. All percentages are by weight. The percentages indicated in the Tables are percentages by weight of the total composition.

Example 1, as detailed in Table 1, was prepared in the following manner. A 20% aqueous solution of the Gantrez S-95 co-polymer was slowly added to a 50% aqueous solution of the ACH, whilst stirring at 8000 rpm using a Silverson L4RT laboratory scale mixer. The addition was performed at room temperature and resulted in a rise in temperature. Stirring was continued for 10 minutes after the addition was complete and the resulting viscous liquid/gel mixture was then allowed to cool back to room temperature.

The Volpo S-2, Brij 721, and sunflower oil were heated to 85°C. In a separate vessel, water was heated to the same temperature. Whilst stirring at 4500 rpm, the water was added to the oil/surfactant mixture, maintaining the temperature at 85°C. Stirring at 4500 rpm was continued at 85°C for 10 minutes and then whilst cooling the mixture to 35°C. The viscous liquid/gel mixture prepared from the ACH and the Gantrez S-95 co-polymer was then added whilst stirring at 4500 rpm. Following cooling to 25°C, the perfume was added and mixture stirred for a further two minutes. Comparative example A was prepared in an analogous manner to Example 1, but without the addition of the Gantrez S-95 to the ACH solution.

**Table 1**

| **Raw material** | **Trade name** | **Example 1** | **Example A** |
|---|---|---|---|
| Aluminium chlorohydrate | Chlorhydrol¹ | 17.5 | 17.5 |
| MA/MVE co-polymer | Gantrez S-95² | 2.0 | -- |
| Steareth-2 | Volpo S-2³ | 2.6 | 2.6 |
| Steareth-21 | Brij 721⁴ | 0.6 | 0.6 |
| Sunflower oil | | 4.0 | 4.0 |
| Perfume | | 0.8 | 0.8 |
| Water | | To 100 | To 100 |

| | | | |
|---|---|---|---|
| 1. *Ex* Reheis. Added as a 50% aqueous solution to give 17.5% of ACH solids in the final products. | | | |
| 2. *Ex* International Speciality Products, Inc. Added as a 20% aqueous solution to give 2.0% of co-polymer solids in the final product. | | | |
| 3. *Ex* Croda. | | | |
| 4. *Ex* Uniqema. | | | |

Using standard 'hot room' evaluation protocols, the underarm antiperspirancy performance of Examples 1 and A were compared, using panels of at least 30 female volunteers. In a first test, the average sweat weight reduction resulting from the use of Example 1 was 17% greater than that resulting from the use of Example A. In a second test, the average sweat weight reduction resulting from the use of Example 1 was 18% greater than that resulting from the use of Example A. Both of these results were significant at the 95% level.

The Sauter mean particle size (D[3,2]) of a liquid/gel mixture prepared from a 50% ACH solution and a 20% Gantrez S-95 co-polymer solution, prepared as described above, was measured to be 17.2 microns using a Malvern Mastersizer. This composition was found to have storage stability far superior to two other compositions of the same components at the same levels having Sauter mean particle sizes (D[3,2]) of 45.3 microns and 60.5 microns; these latter compositions being prepared by using lesser amounts of shear. The higher particle size mixtures were unsuitable for use in commercial antiperspirant compositions, due to their instability.

The compositions detailed in Table 2 may also be prepared in accordance with the invention, using methods analogous to that used in the preparation of Example 1.

**Table 2**

| **Raw material** | **Trade name** | **Example 2** | **Example 3** | **Example 4** |
|---|---|---|---|---|
| Aluminium chlorohydrate | Chlorhydrol¹ | 15 | 17.5 | 20 |
| MA/MVE co-polymer | Gantrez S-95¹ | 0.5 | -- | -- |
| MAA/MA/MVE co-polymer | Gantrez AN-119² | -- | 2.0 | 1.0 |
| Glycerol | | 1.0 | -- | -- |
| Propylene glycol | | -- | 1.0 | -- |
| Steareth-2 | Volpo S-2¹ | 2.6 | 2.5 | 2.5 |
| Steareth-21 | Brij 721¹ | 0.6 | 0.5 | 0.6 |
| Isopropyl myristate | | 3.0 | -- | -- |
| C12-C15 alcohol benzoate | Finsolv TN³ | -- | 2.0 | -- |
| PPG-14 butyl ether | Fluid AP⁴ | -- | -- | 2.5 |
| Perfume | | 0.7 | 0.5 | 0.9 |
| Water | | To 100 | To 100 | To 100 |

| | | | | |
|---|---|---|---|---|
| 1. As in Table 1. | | | | |
| 2. ex International Speciality Products Inc. Amounts indicated are of polymer solids. | | | | |
| 3. ex Finetex, Inc. | | | | |
| 4. ex Amerchol Corp. | | | | |

## Claims

1. An antiperspirant composition comprising an aqueous phase, an antiperspirant salt and a polymer having Bronsted acid groups, **characterised in that** the antiperspirant salt and polymer are suspended in the aqueous phase and the composition has a Sauter mean particle size (D[3,2]) of 30 microns or less.

2. An antiperspirant composition according to claim 1, wherein the polymer acts a co-gellant for the antiperspirant salt.

3. An antiperspirant composition according to claim 2, wherein the antiperspirant salt and the polymer exist as particles of co-gel suspended in the aqueous phase.

4. An antiperspirant composition according to claim 3, wherein the antiperspirant salt and the polymer exist as particles of co-gel having a Sauter mean particle size (D[3,2]) of 30 microns or less.

5. An antiperspirant composition according to any of preceding claims, wherein the polymer is water soluble at pH7, having a solubility at 37°C of 10 g/l or greater.

6. An antiperspirant composition according to any of preceding claims, wherein the aqueous phase is a continuous phase.

7. An antiperspirant composition according to claim 6, having emulsified oil droplets and particles of AP salt-polymer co-gel suspended therein.

8. An antiperspirant composition according to any of preceding claims, wherein the antiperspirant salt is present at a level of from 5% to 35% by weight of the composition.

9. An antiperspirant composition according to any of preceding claims, wherein the polymer is present at a level of from 0.05% to 5% by weight of the composition.

10. An antiperspirant composition according to any of preceding claims, wherein the weight ratio of the AP salt to the polymer is 50:1 or less.

11. An antiperspirant composition according to any of preceding claims, comprising a perfume at a level of from 0.7% to 1.7% by weight of the composition.

12. A product comprising a composition as described in any of the preceding claims and an applicator suitable for roll-on application of the composition.

13. A cosmetic method of achieving an antiperspirancy and/or deodorancy benefit, said method comprising the application to the human body of a composition as described in any of claims 1 to 11.

14. A method for the manufacture of an antiperspirant composition, said method comprising the suspension in an aqueous phase of an antiperspirant salt and a polymer having Bronsted acid groups, the composition being sheared to give a Sauter mean particle size (D[3,2]) of 30 microns or less.

## Patentansprüche

1. Schweißhemmende Zusammensetzung, umfassend eine wässerige Phase, ein schweißhemmendes Salz und ein Polymer mit Brönsted-Säuregruppen, **dadurch gekennzeichnet, daß** das schweißhemmende Salz und das Polymer in der wässerigen Phase suspendiert sind und die Zusammensetzung eine mittlere Sauter-Teilchengröße (D[3,2]) von 30 Mikrometern oder weniger hat.

2. Schweißhemmende Zusammensetzung nach Anspruch 1, wobei das Polymer als Gelierhilfsmittel für das schweißhemmende Salz agiert.

3. Schweißhemmende Zusammensetzung nach Anspruch 2, wobei das schweißhemmende Salz und das Polymer als Co-Gelteilchen suspendiert in der wässerigen Phase vorliegen.

4. Schweißhemmende Zusammensetzung nach Anspruch 3, wobei das schweißhemmende Salz und das Polymer als Co-Gelteilchen mit einer mittleren Sauter-Teilchengröße (D[3,2]) von 30 Mikrometern oder weniger vorliegen.

5. Schweißhemmende Zusammensetzung nach einem der vorhergehenden Ansprüche, wobei das Polymer bei pH 7 wasserlöslich ist und bei 37 °C eine Löslichkeit von 10 g/l oder mehr aufweist.

6. Schweißhemmende Zusammensetzung nach einem der vorhergehenden Ansprüche, wobei die wässerige Phase eine kontinuierliche Phase ist.

7. Schweißhemmende Zusammensetzung nach Anspruch 6, die emulgierte Öltröpfchen und darin suspendierte AP-Salz-Polymer-Co-Gelteilchen aufweist.

8. Schweißhemmende Zusammensetzung nach einem der vorhergehenden Ansprüche, wobei das schweißhemmende Salz bei einem Gehalt von 5 bis 35 Gew.-% der Zusammensetzung vorliegt.

9. Schweißhemmende Zusammensetzung nach einem der vorhergehenden Ansprüche, wobei das Polymer bei einem Gehalt von 0,05 bis 5 Gew.-% der Zusammensetzung vorliegt.

10. Schweißhemmende Zusammensetzung nach einem der vorhergehenden Ansprüche, wobei das Gewichtsverhältnis des AP-Salzes zu dem Polymer 50 : 1 oder weniger beträgt.

11. Schweißhemmende Zusammensetzung nach einem der vorhergehenden Ansprüche, umfassend einen Duftstoff bei einem Gehalt von 0,7 bis 1,7 Gew.-% der Zusammensetzung.

12. Produkt, umfassend eine Zusammensetzung wie in einem der vorhergehenden Ansprüche beschrieben, und einen Applikator, der zur Deoroller-Applikation der Zusammensetzung geeignet ist.

13. Kosmetisches Verfahren zum Erhalt von Schweißhemmung und/oder Geruchsbeseitigung, wobei das Verfahren die Auftragung einer Zusammensetzung, wie in einem der Ansprüche 1 bis 11 beschrieben, auf den menschlichen Körper umfaßt.

14. Verfahren zur Herstellung einer schweißhemmenden Zusammensetzung, wobei das Verfahren die Suspension eines schweißhemmenden Salzes und eines Polymers mit Brönsted-Säuregruppen in einer wässerigen Phase umfaßt, wobei die Zusammensetzung zum Erhalt einer mittleren Sauter-Teilchengröße (D[3,2]) von 30 Mikrometern oder weniger geschert wird.

## Revendications

1. Composition anti-transpirante comprenant une phase aqueuse, un sel anti-transpirant et un polymère portant des groupes acides de Brönsted, **caractérisée en ce que** le sel anti-transpirant et le polymère sont en suspension dans la phase aqueuse et la composition a une taille de particule moyenne de Sauter (D[3,2]) de 30 microns ou moins.

2. Composition anti-transpirante selon la revendication 1, dans laquelle le polymère agit comme co-gélifiant pour le sel anti-transpirant.

3. Composition anti-transpirante selon la revendication 2, dans laquelle le sel anti-transpirant et le polymère existent sous forme de particules de co-gel en suspension dans la phase aqueuse.

4. Composition anti-transpirante selon la revendication 3, dans laquelle le sel anti-transpirant et le polymère existent sous forme de particules de co-gel ayant une taille de particule moyenne de Sauter (D[3,2]) de 30 microns ou moins.

5. Composition anti-transpirante selon l'une quelconque des revendications précédentes, dans laquelle le polymère est hydrosoluble à pH 7, en ayant une solubilité à 37 °C de 10 g/l ou plus.

6. Composition anti-transpirante selon l'une quelconque des revendications précédentes, dans laquelle la phase aqueuse est une phase continue.

7. Composition anti-transpirante selon la revendication 6, possédant des gouttelettes d'huile émulsifiée et des particules de co-gel de sel AT-polymère en suspension dans celles-ci.

8. Composition anti-transpirante selon l'une quelconque des revendications précédentes, dans laquelle le sel anti-transpirant est présent à une teneur allant de 5 % à 35 % du poids de la composition.

9. Composition anti-transpirante selon l'une quelconque des revendications précédentes, dans laquelle le polymère est présent à une teneur allant de 0,05 % à 5 % du poids de la composition.

10. Composition anti-transpirante selon l'une quelconque des revendications précédentes, dans laquelle le rapport massique de sel AT au polymère est de 50:1 ou moins.

11. Composition anti-transpirante selon l'une quelconque des revendications précédentes, comprenant un parfum à une teneur allant de 0,7 % à 1,7 % du poids de la composition.

12. Produit comprenant une composition telle que décrite dans l'une quelconque des revendications précédentes et un applicateur adapté à une application de la composition via une bille.

13. Méthode cosmétique pour atteindre un gain de pouvoir anti-transpirant et/ou de pouvoir déodorant, ladite méthode comprenant l'application au corps humain d'une composition telle que décrite dans l'une quelconque des revendications 1 à 11.

14. Méthode de fabrication d'une composition anti-transpirante, ladite méthode comprenant la mise en suspension dans une phase aqueuse d'un sel anti-transpirant et d'un polymère portant des groupes acides de Brönsted, la composition étant soumise à un cisaillement pour donner une taille de particule moyenne de Sauter (D[3,2]) de 30 microns ou moins.
